# FASCICULE DE BREVET EUROPEEN

(11) **EP 2 376 055 B1**
(45) Date de publication et mention de la délivrance du brevet: **24.04.2013**
(21) Numéro de dépôt: 09799108.7
(22) Date de dépôt: 23.12.2009
(51) Int. Cl.: A61K 8/33, A61K 8/97, A61Q 5/00, A61Q 19/00

(54) **Composition cosmétique comprenant un hydrolysat de gomme de caroube**
Kosmetische Zusammensetzung mit einem Johannisbrotgumhydrolysat
Cosmetic composition containing a locust bean gum hydrolysate

(30) Priorité: 23.12.2008 FR 0859025
(43) Date de publication de la demande: 19.10.2011
(73) Titulaire: Pierre Fabre Dermo-Cosmétique, 92100 Boulogne-Billancourt (FR)
(72) Inventeur: FABRE, Bernard, F-31450 Belberaud (FR); FIORINI-PUYBARET, Christel, F-31240 L'union (FR)
(74) Mandataire: Regimbeau
(86) Numéro de dépôt international: PCT/EP2009/067831
(87) Numéro de publication internationale: WO 2010/072801

(56) Documents cités:
- EP-A1- 0 602 991
- WO-A1-2008/076461
- FR-A1- 2 810 324
- US-A1- 2008 064 064

## Description

L'invention se rapporte à une composition cosmétique capillaire comprenant un hydrolylat de gomme de caroube.

La caroube est le fruit du caroubier, *Ceratonia siliqua L.,* plante dioïque de la famille des fabacées, originaire des régions méditerranéennes (Afrique du Nord, Proche-Orient, Europe méridionale) et des îles Canaries. La gomme de caroube, appelée également farine de grains de caroubiers, est obtenue par broyage de l'endosperme des grains de caroubier, contenus dans les gousses. Les graines de caroube sont brunes, de forme ovoïde aplatie, biconvexes et très dures. Elles sont séparées les unes des autres par des cloisons pulpeuses. On en compte 15 à 20 par gousse. Les gousses sont aussi appelées « caroubes ». Elles sont pendantes, de 10 à 30 cm de long sur 1,5 à 3 cm de large, d'abord de couleur verte, puis brun foncé à maturité.

La production de la gomme de caroube exige donc une séparation optimale des graines de la gousse d'une part, puis de l'endosperme des autres parties de la graine, l'endocarpe et le germe, d'autre part. La gomme de caroube consiste essentiellement en un polysaccharide de type galactomannane dont la masse moléculaire s'élève de 50 000 à quelques millions. La gomme de caroube forme avec l'eau un colloïde qui atteint son optimum de solubilisation quand on porte l'eau à une température de 80 à 90°C. Les solutions obtenues sont légèrement troubles et possèdent une très haute viscosité.

Cette gomme est surtout connue dans l'industrie alimentaire comme épaississant et gélifiant dans des soupes, sauces, crèmes, glaces, etc. L'industrie pharmaceutique l'utilise également pour ces mêmes propriétés. EP-602 991 décrit l'utilisation d'un hydrolysat de gomme de caroube comme gélifiant.

Du fait de son haut pouvoir gélifiant et viscosant, il est très difficile d'utiliser la gomme de caroube dans une formule cosmétique fluide comme un shampoing. De plus, du fait de son haut poids moléculaire, la gomme de caroube appliquée sur des cheveux aurait un effet alourdissant inesthétique et non désirable en cosmétique. Malgré ces effets négatifs, et du fait de sa structure chimique et de son affinité pour les cheveux, la gomme de caroube présente des propriétés intéressantes telles que son effet gainant et protecteur envers les cheveux.

Il existe toute une gamme de produits ajoutés au shampoing ou à l'après shampoing permettant de conditionner les cheveux et de leur apporter des caractéristiques nouvelles que l'on ne trouve pas dans un shampoing de base, que ce soit un effet de protection, un effet stylisant, ou l'apport d'une couleur particulière, par exemple. Ainsi, l'utilisation de dérivés de polysaccharides dans des produits capillaires est connue. Leurs caractéristiques physico-chimiques (viscosité, solubilité, couleur, etc...) leur permettent plus ou moins facilement d'être incorporés dans lesdites compositions capillaires. Les produits à longues chaînes polysaccharidiques d'extraits naturels sont difficilement solubles en tant que tels dans les compositions cosmétiques, et doivent généralement subir une intervention humaine par l'outil chimique par exemple afin de les rendre utilisables.

Le demandeur a donc cherché à modifier la gomme de caroube afin de surmonter les inconvénients cités ci-dessus comme l'effet alourdissant et conserver le bénéfice gainant et protecteur de cette gomme envers les cheveux.

Ainsi, le demandeur a préparé de façon surprenante un hydrolysat de gomme de caroube présentant de nouvelles propriétés physico-chimiques lui permettant d'être incorporé dans un shampoing. Le demandeur a alors constaté un effet volumateur des cheveux notoire et inattendu de cet hydrolysat.

L'invention a ainsi pour objet un hydrolysat de gomme de caroube présentant une teneur en sucres réducteurs comprise entre 10% et 60%, avantageusement entre 25% et 45% en poids par rapport au poids de la matière sèche dudit hydrolysat.

Un hydrolysat est un produit issu d'une réaction d'hydrolyse d'un substrat, ici de gomme de caroube. Une hydrolyse est une décomposition d'un corps par fixation des ions H⁺ et OH- provenant de la dissociation de l'eau. Ainsi, la présence d'eau est nécessaire pour que l'hydrolyse se fasse. Le substrat peut être dissous dans de l'eau pure ou dans un mélange d'eau et un ou plusieurs autres solvants.

Un sucre réducteur est un sucre comportant une fonction aldéhyde terminal donneuse d'électrons dans une réaction d'oxydo-réduction. Historiquement, ce terme vient de la découverte de Fehling au 19ème siècle qui prouva que certains sucres réagissaient avec des ions cuivriques pour les transformer en ions cuivreux. Un tel dosage permet de quantifier les fonctions terminales d'un sucre. Le dosage des sucres réducteurs permet de mesurer l'efficacité d'une hydrolyse et de doser les oligomères générés.

Les meilleurs résultats de l'effet volumateur ont été remarqués lorsque les teneurs en sucres réducteurs de l'hydrolysat de gomme de caroube se situent dans une gamme comprise entre 10% et 60%, et meilleurs encore dans une gamme de 25 à 45% en poids par rapport au poids de la matière sèche dudit hydrolysat.

La gomme de caroube est essentiellement constituée d'un polygalatomannane caractéristique, comprenant une longue chaîne fondamentale composée uniquement de mannoses liés par des liaisons β 1-4-glycosidiques. Cette chaîne porte des ramifications constituées d'une seule unité de galactose liée à la chaîne de mannoses par des liaisons α-glycosidiques. Ce polysaccharide est constitué de mannose et de galactose dans une proportion naturelle située approximativement à 4:1. On peut supposer que l'hydrolyse va donc se porter essentiellement sur ce polysaccharide, mais également sur les composés ou traces de composés accompagnant ce polysaccharide dans la gomme de caroube.

L'hydrolysat de gomme de caroube selon l'invention peut être un hydrolysat chimique et/ou physique et/ou biologique, c'est-à-dire issu de procédés chimiques et/ou physiques et/ou biologiques.

Dans un mode de réalisation particulier, l'hydrolysat de gomme de caroube selon l'invention est un hydrolysat enzymatique avantageusement obtenu par une enzyme de type mannase.

Les enzymes de type mannase sont des hydrolases, plus spécifiquement, des oxidases. Elles vont donc être susceptibles d'hydrolyser la liaison osidique dans le motif « mannose-mannose » situé dans des fragments polysaccharidiques ou toute autre molécule comprenant ce motif. Ainsi, l'action de la mannase sur la gomme de caroube va produire des polysaccharides de tailles réduites par rapport au galactomannane initial.

L'hydrolysat de gomme de caroube peut se présenter sous la forme d'un extrait sec, en particulier sous forme de poudre ou d'un quelconque solide, ou d'un extrait liquide fluide ou visqueux.

On appelle extrait sec d'hydrolysat de gomme de caroube, l'ensemble de toutes les substances qui ne se volatilisent pas et sont issues de la gomme de caroube hydrolysée.

On appelle extrait liquide d'hydrolysat de gomme de caroube, l'ensemble de toutes les substances issues de l'hydrolyse de la gomme de caroube en solution inorganique ou organique.

L'hydrolysat de gomme de caroube est préparé par un procédé présentant les étapes suivantes :
a) hydrolyse de la gomme de caroube. L'hydrolyse se fait dans l'eau.
b) interruption de l'hydrolyse lorsque la teneur en sucres réducteurs est comprise entre 10% et 60%, avantageusement entre 25% et 45% en poids par rapport au poids de la matière sèche dudit hydrolysat.
c) séparation solide/liquide du mélange obtenu à l'étape b) pour obtenir un hydrolysat de gomme de caroube.

Avantageusement, l'étape c) de séparation est réalisée par filtration et/ou centrifugation.

Le procédé peut se faire à l'étape a) par une hydrolyse par voie physique et/ou chimique et/ou biologique.

Plusieurs techniques peuvent produire un hydrolysat de gomme de caroube. Une source d'eau est obligatoire afin d'obtenir un hydrolysat. Par exemple, une hydrolyse chimique peut se faire grâce à un acide fort.

Une hydrolyse physique peut se faire par ultrafiltration.

Une hydrolyse biochimique peut se faire avec une enzyme.

Une hydrolyse biologique peut se faire avec un organisme vivant tel qu'un microorganisme.

Le résultat final de tous ces procédés est un hydrolysat de gomme de caroube caractérisable par sa teneur en sucres réducteurs.

La mesure du taux de sucres réducteurs peut se faire de toute manière classique bien connue de l'homme du métier. Par exemple on peut réaliser cela par un dosage par l'acide 3,5-dinitrosalicylique (DNS), encore appelé acide 2-hydroxy-3,5-dinitrobenzoïque. Le DNS est réduit en acide 3-amino-5-nitrosalicylique par le sucre réducteur selon la réaction suivante :

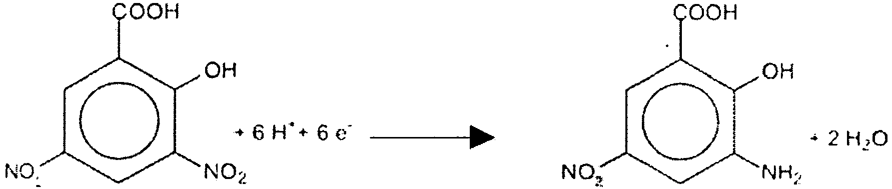

Le sucre est quant à lui oxydé en divers produits d'oxydations :

Sucre → Produits d'oxydation + n.e⁻

L'acide 3-amino-5-nitrosalicylique est un composé rouge. Cette réaction n'est pas stoechiométrique : il n'y a pas de bilan d'oxydoréduction. L'intensité de la coloration rouge est proportionnelle à la concentration de la fonction aldéhyde terminal si l'on opère dans des conditions physico-chimiques constantes. Les droites d'étalonnage ne passent pas toujours par l'origine.

L'interruption de l'hydrolyse de gomme de caroube est propre à la technique d'hydrolyse choisie : arrêt du phénomène physique utilisé, arrêt du phénomène chimique par neutralisation du pH, dénaturation de l'enzyme mise en jeu par élévation de température ou ajout d'alcool, stérilisation de l'hydrolysat.

L'hydrolysat de gomme de caroube peut alors être filtré sur une membrane ou un fritté.

L'hydrolysat peut aussi être centrifugé afin de récupérer un surnageant limpide, exempt de particules.

Le procédé peut comprendre en outre une étape ultérieure d) de concentration de l'hydrolysat obtenu à l'étape c).

Celle-ci s'effectue par une quelconque technique d'évaporation de l'eau utilisée lors de l'hydrolyse. Ainsi, elle peut se faire par une mise sous vide plus ou moins prononcée avec une éventuelle hausse de la température de l'hydrolysat ou par toute autre méthode bien connue de l'homme du métier.

Le procédé peut également comprendre en outre une étape d'ajout de conservateur microbien et/ou d'ajout de solvant(s) parmi la glycérine, le propylène glycol, le butylène glycol, l'éthanol ou de tout solvant permettant une propriété microbiologique. Cette étape peut se faire avant concentration ou après une concentration partielle. Les quantités de solvant à ajouter peuvent varier selon leurs propriétés anti-microbiennes.

Dans le cas d'un conservateur microbien ajouté directement à la solution aqueuse d'hydrolyse, ce conservateur microbien peut être choisi dans la liste suivante : le chlorure de cétrimonium, l'éthanol, le capryl glycol, la glycérine, par exemple. Ces ajouts de solvants ou de conservateurs peuvent s'effectuer à n'importe quelle étape décrite. Les extraits ainsi obtenus ont perdu leur propriété colloïdale et peuvent être miscibles à l'eau et/ou dilués avec de l'eau.

L'hydrolysat peut également se présenter sous une forme solide, tel qu'un extrait sec. Le procédé comprend alors en outre une étape ultérieure e) de séchage de l'hydrolysat pour obtenir un extrait sec d'hydrolysat de gomme de caroube. Dans un premier temps, l'hydrolysat est concentré puis séché dans une étuve sous vide par exemple avec une température comprise entre 40 et 90°C, ou par nébulisation, par lyophilisation ou toute autre méthode. Les extraits ainsi obtenus ont perdu leur propriété colloïdale et sont très solubles dans l'eau.

Selon un mode de réalisation préféré, l'étape a) du procédé de préparation d'un hydrolysat de gomme de caroube selon l'invention est réalisé par une hydrolyse enzymatique, avantageusement par une enzyme de type mannase.

Avantageusement, dans le cadre de ce mode de réalisation préféré, l'étape a) du procédé de fabrication consiste à mettre en contact dans une solution aqueuse de la gomme de caroube dans des conditions de température et de pH adéquat avec une enzyme de type mannase. Ainsi, on solubilise dans l'eau initialement l'enzyme sous agitation constante. L'eau est préalablement portée à une température pouvant varier entre 30°C et 70°C, préférentiellement entre 40°C et 50 °C. Les proportions d'eau par rapport à l'enzyme varient dans un rapport eau/enzyme (poids/poids) de 10/1 à 1000/1 et préférentiellement de 20/1 à 200/1. La gomme de caroube est alors ajoutée à la solution dans un rapport eau/gomme (poids/poids) pouvant varier de 5/1 à 20/1, préférentiellement de 7/1 à 12/1. Ainsi, le procédé de préparation d'un hydrolysat enzymatique de gomme de caroube peut présenter un rapport réactionnel entre la gomme de caroube et l'enzyme de type mannase lors de l'étape a) compris entre 1/2 et 200/1 en poids sec.

Le pH peut alors être ajusté par ajout d'acide (HCl, H₂SO₄, CH₃COOH par exemple) ou de base (KOH, NaOH par exemple) de façon à atteindre des valeurs situées entre 2 à 7 et préférentiellement entre 4 et 5.

L'agitation et la température sont maintenues tout le long de l'hydrolyse enzymatique pendant 30 minutes à 10 h et préférentiellement entre 1h30 et 3h. Lorsque la teneur en sucres réducteurs atteint la valeur voulue, l'hydrolyse peut être interrompue. Dans le cadre de ce mode de réalisation préféré, le procédé faisant intervenir une hydrolyse enzymatique à l'étape a), est de préférence interrompu à l'étape b) par une élévation de la température entre 70 et 100°C, avantageusement entre 95 et 100°C, pendant une durée variant de 5 minutes à 30 minutes, ou par l'ajout d'un alcool.

L'invention concerne une composition cosmétique comprenant, à titre de principe actif, un hydrolysat de gomme de caroube.

Ainsi, la composition cosmétique de l'invention comprend, à titre de principe actif, un hydrolysat de gomme de caroube avec une teneur en sucres réducteurs comprise entre 10% et 60% en poids par rapport au poids de la matière sèche dudit hydrolysat, préférentiellement avec une teneur en sucres réducteurs comprise entre 25% et 45% en poids par rapport au poids de la matière sèche dudit hydrolysat.

De manière avantageuse, la composition cosmétique de l'invention comprend, à titre de principe actif, un hydrolysat de gomme de caroube de l'invention, enzymatique, préférentiellement obtenu par une enzyme de type mannase.

L'hydrolysat de gomme de caroube de l'invention peut se présenter dans la composition cosmétique de l'invention sous la forme d'un extrait sec ou d'un extrait liquide.

Préférentiellement, la composition cosmétique selon la présente invention comprend une quantité d'extrait sec d'hydrolysat de gomme de caroube selon l'invention, comprise entre 0,1 g et 2,0 g, et préférentiellement entre 0,1 g et 1,0 g pour 100 g de ladite composition et de manière particulièrement préférée d'environ 0,5 g pour 100 g de composition cosmétique.

La composition cosmétique selon la présente invention peut avantageusement se présenter sous toutes les formes normalement utilisées dans le domaine cosmétique pour une application topique. Ainsi et préférentiellement, elle peut être sous la forme d'un shampoing, d'un gel, d'une lotion, d'une mousse, d'un spray, d'une dispersion, d'un sérum, de masque, de lait corporel, baume amplifiant ou de crème par exemple.

De manière préférentielle, la composition cosmétique selon invention est une composition capillaire.

Par composition capillaire, on entend une composition aqueuse et/ou organique destinée au lavage, à la coloration, au soin ou au coiffage des cheveux. Avantageusement, elle fixe la coiffure et/ou apporte un effet bénéfique sur l'état cosmétique des cheveux et/ou apporte de la brillance aux cheveux, et/ou permet le maintien ou la mise en forme de la coiffure.

Les compositions conformes à l'invention peuvent être utilisées pour le lavage ou le traitement des matières kératiniques telles que les cheveux, les cils, les sourcils, le cuir chevelu et plus particulièrement les cheveux.

La composition cosmétique selon l'invention comprend, en outre, des excipients usuels cosmétiquement compatibles.

Les excipients usuels compatibles à la composition cosmétique capillaire de l'invention peuvent être tout excipient connu de l'homme du métier en vue d'obtenir une composition cosmétique pour une application topique sous les formes telles que décrites ci-dessus.

La composition cosmétique selon l'invention peut en particulier contenir des additifs et aides à la formulation, tels que des tensio-actifs de type émulsionnant, nettoyant, moussant, etc..., des agents complexants, des épaississants, des gélifiants, des stabilisants, des conservateurs dont des antimicrobiens et des antioxydants, des conditionneurs, des acidifiants, des alcalinisants, des émollients, des solvants, des colorants, des parfums.

La composition capillaire de l'invention peut comprendre d'autres composés utiles au conditionnement du cheveu, tels que des agents de coloration ou de brillance.

Un autre objet de l'invention est l'utilisation d'un hydrolysat de gomme de caroube selon l'invention, comme agent cosmétique.

Ainsi, un hydrolysat de gomme de caroube caractérisé par une teneur en sucres réducteurs comprise entre 10% et 60% en poids par rapport au poids de la matière sèche dudit hydrolysat, préférentiellement par une teneur en sucres réducteurs comprise entre 25% et 45% en poids par rapport au poids de la matière sèche dudit hydrolysat, est utilisé comme agent cosmétique.

De manière avantageuse, l'hydrolysat de gomme de caroube de l'invention utilisé comme agent cosmétique, est un hydrolysat enzymatique, préférentiellement obtenu par une enzyme de type mannase.

En outre, un hydrolysat de gomme de caroube de l'invention peut être utilisé comme agent cosmétique sous la forme d'un extrait sec ou d'un extrait liquide.

Avantageusement, l'hydrolysat de gomme de caroube de la présente invention est utilisé comme agent cosmétique dans une composition cosmétique, de préférence capillaire.

Un autre objet de l'invention est l'utilisation d'une composition capillaire selon l'invention pour donner un effet volumateur aux cheveux.

Un autre objet de l'invention est l'utilisation d'une composition capillaire selon l'invention pour gainer les cheveux en les protégeant, avantageusement pour une application sur les cheveux fins et/ou sans volume.

La composition capillaire selon l'invention est destinée à créer du volume des racines jusqu'aux pointes des cheveux. Les cheveux sont alors parfaitement galbés pour un volume longue tenue. La texture du cheveu se trouve ainsi améliorée.

Les exemples suivants illustrent l'invention sans en limiter la portée.

### Exemple 1 : conditions d'hydrolyse enzymatique de la gomme de caroube par une enzyme de type mannase.

1000 litres d'eau sont portés dans un réacteur à une température de 45° C sous agitation. 10 kg de mannase sont ajoutés. Après dissolution on ajoute 100 kg de farine de caroube. Le pH est ajusté à 4,5.

La température et l'agitation sont maintenues pendant 3 heures puis la température est portée à 95° C pendant 10 minutes. Après refroidissement on procède à une séparation solide/liquide sur filtre. La solution recueillie est concentrée puis séchée. On obtient ainsi 80 kg de gomme de caroube hydrolysée dont la teneur en sucres réducteurs est de 35 % environ (poids/poids).

### Exemple 2 : conditions d'hydrolyse enzymatique de la gomme de caroube par une enzyme de type mannase

100 litres d'eau sont portés dans un réacteur à une température de 50° C sous agitation. On ajoute 0,5 kg de mannase puis 20 kg de gomme de caroube. Le pH est ajusté à 4. L'hydrolyse est maintenue pendant 2 heures, puis la température est portée à 95° C pendant 15 minutes. Après refroidissement, la solution est filtrée puis on ajoute au filtrat un égal volume de propylène glycol. Les dosages des sucres réducteurs indiquent une valeur par rapport à la matière sèche de 20 % environ (poids/poids).

### Exemple 3 : étude d'efficacité de l'hydrolysat de gomme de caroube en cosmétique capillaire.

Une étude d'efficacité en cosmétique capillaire a permis d'évaluer l'activité d'une solution aqueuse à 1 % de la gomme de caroube hydrolysée obtenue par le procédé décrit dans l'exemple 1 sur le volume des mèches de cheveux en comparaison avec un témoin non traité. Pour cela, les mèches sont préalablement lavées de façon standardisée puis séchées également de façon standardisée. Ces mèches sont ensuite immergées dans la solution aqueuse de gomme de caroube hydrolysée ou dans de l'eau pendant 3 minutes. Les mèches sont alors séchées pendant 18 heures sur un moule en forme de « cage d'oiseau ». L'activité sur le volume des cheveux est mesurée par analyse d'image. Pour cela les mèches sont placées face à une source lumineuse et subissent une rotation de 180°. Au cours de cette rotation, le volume des mèches en mouvement est projeté sur un écran et leur surface est mesurée. Plus la surface est grande, plus l'effet volumateur des produits testés est grand. L'analyse des résultats met en évidence une différence significative du volume des mèches de + 11 % entre les 2 groupes en faveur du groupe traité par la gomme de caroube hydrolysée, et justifie ainsi de son utilisation dans un shampoing volumateur.

### Exemple 4 : composition : shampoing volumateur.

| | |
|---|---|
| Extrait sec d'hydrolysat de gomme de caroube | 0,3 à 1,0 g |
| Lauryl sulfate d'ammonium | 1,5 g |
| Laureth sulfate de sodium | 10,0 g |
| Cocoamphodiacétate | 10,0 g |
| Ceteareth-60 Myristyl glycol | 0,75 g |
| EDTA disodique | 0,2 g |
| Hydroxypropyl guar | 0,25 g |
| Capryl glycol | 1,0 g |
| Chlorure de cétrimonium | 2,0 g |
| Acide citrique monohydraté | qsp pH = 5 |
| Colorant(s) | qs. |
| Parfum | qs. |
| Eau purifiée | qsp 100g |

### Exemple 5 : composition : gelée sprayable sans rinçage volumatrice

| | |
|---|---|
| Extrait sec d'hydrolysat de gomme de caroube | 0,1 à 0,7 g |
| Gomme Xanthane | 0,3 g |
| Copolymère Vinylpyrrolidone / vinylacrylate | 1,0 g |
| Dimethicone copolyol | 0,3 g |
| Huile de ricin | 0,3 g |
| Alcool éthylique dénaturé | 6,0 g |
| EDTA disodique | 0,2 g |
| Parfum | qs |
| Conservateur(s) | qs |
| Hydroxyde de sodium | qsp pH = 5,5 - 6,5 |
| Eau purifiée | qsp 100 g |

### Exemple 6 : composition : mousse volumatrice

| | |
|---|---|
| Extrait sec d'hydrolysat de gomme de caroube | 0,1 à 0,7 g |
| Copolymère Vinylpyrrolidone / vinylacrylate | 1,0 g |
| Chlorure de cétrimonium | 1,0 g |
| Polyvinyl pyrrolidone - acrylamide quaternisé | 3,0 g |
| Glycérine | 0,5 g |
| Panthenol | 0,2 g |
| Ceteareth-20 | 0,2 g |
| Hydroxyéthylcellulose | 0,1 g |
| Parfum | qs |
| Acide citrique monohydraté | qsp pH = 5 - 6 |
| Eau purifiée | qsp 100g |

## Revendications

1. Composition cosmétique comprenant, à titre de principe actif, un hydrolysat de gomme de caroube **caractérisé par** une teneur en sucres réducteurs comprise entre 10% et 60% en poids par rapport au poids de la matière sèche dudit hydrolysat.

2. Composition cosmétique selon la revendication 1, **caractérisé en ce que** l'hydrolysat de gomme de caroube comprend une teneur en sucres réducteurs entre 25% et 45% en poids par rapport au poids de la matière sèche dudit hydrolysat.

3. Composition cosmétique selon l'une quelconque des revendications 1 ou 2, **caractérisé en ce que** l'hydrolysat de gomme de caroube est un hydrolysat enzymatique.

4. Composition cosmétique selon la revendication 3, **caractérisé en ce qu'**il s'agit d'un hydrolysat enzymatique obtenu par une enzyme de type mannase.

5. Composition cosmétique selon l'une quelconque des revendications précédentes, **caractérisé en ce que** l'hydrolysat de gomme de caroube est un hydrolysat se présentant sous la forme d'un extrait sec ou d'un extrait liquide.

6. Composition cosmétique selon l'une quelconque des revendications précédentes comprenant une quantité d'hydrolysat de gomme de caroube comprise entre 0,1 g et 2,0 g pour 100 g de ladite composition.

7. Composition cosmétique selon l'une quelconque des revendications précédentes, se présentant sous la forme d'un shampoing, d'un gel, d'une lotion, d'une mousse, d'un spray, d'une dispersion, d'un sérum, d'un masque, d'un lait corporel, d'un baume amplifiant ou d'une crème.

8. Composition capillaire selon l'une quelconque des revendications précédentes.

9. Utilisation d'un hydrolysat de gomme de caroube **caractérisé par** une teneur en sucres réducteurs comprise entre 10% et 60% en poids par rapport au poids de la matière sèche dudit hydrolysat, comme agent cosmétique.

10. Utilisation d'un hydrolysat de gomme de caroube comme agent cosmétique selon la revendication 9, **caractérisé par** une teneur en sucres réducteurs comprise entre 25% et 45% en poids par rapport au poids de la matière sèche dudit hydrolysat.

11. Utilisation d'un hydrolysat de gomme de caroube comme agent cosmétique selon l'une quelconque des revendications 9 ou 10, **caractérisé en ce que** l'hydrolysat de gomme de caroube est un hydrolysat enzymatique.

12. Utilisation d'un hydrolysat de gomme de caroube comme agent cosmétique selon la revendication 11, **caractérisé en ce qu'**il s'agit d'un hydrolysat enzymatique obtenu par une enzyme de type mannase.

13. Utilisation d'un hydrolysat de gomme de caroube comme agent cosmétique selon l'une quelconque des revendications 9 à 12, **caractérisé en ce que** l'hydrolysat de gomme de caroube est un hydrolysat se présentant sous la forme d'un extrait sec ou d'un extrait liquide.

14. Utilisation d'un hydrolysat de gomme de caroube comme agent cosmétique selon l'une quelconque des revendications 9 à 13 dans une composition cosmétique, de préférence capillaire.

15. Utilisation d'une composition capillaire selon la revendication 8, pour donner un effet volumateur aux cheveux.

16. Utilisation d'une composition capillaire selon la revendication 8 pour gainer les cheveux en les protégeant, avantageusement pour une application sur les cheveux fins et/ou sans volume.

## Claims

1. Cosmetic composition comprising, as active ingredient, a locust bean gum hydrolysate **characterised by** a reducing sugars content comprised between 10% and 60% by weight compared to the weight of dry matter of said hydrolysate.

2. Cosmetic composition according to claim 1, **characterised in that** the locust bean gum hydrolysate comprises a reducing sugars content between 25% and 45% by weight compared to the weight of dry matter of said hydrolysate.

3. Cosmetic composition according to any of claims 1 or 2, **characterised in that** the locust bean gum hydrolysate is an enzymatic hydrolysate.

4. Cosmetic composition according to claim 3, **characterised in that** it is an enzymatic hydrolysate obtained by an enzyme of mannase type.

5. Cosmetic composition according to any of the preceding claims, **characterised in that** the locust beangum hydrolysate is a hydrolysate being in the form of a dry extract or a liquid extract.

6. Cosmetic composition according to any of the preceding claims comprising a quantity of locust beangum hydrolysate between 0.1 g and 2.0 g for 100 g of said composition.

7. Cosmetic composition according to any of the preceding claims, being in the form of a shampoo, a gel, a lotion, a foam, a spray, a dispersion, a serum, a mask, a body lotion, a volumising balm or a cream.

8. Hair composition according to any of the preceding claims.

9. Use of a locust bean gum hydrolysate **characterised by** a reducing sugars content comprised between 10% and 60% by weight compared to the weight of dry matter of said hydrolysate as cosmetic agent.

10. Use of a locust bean gum hydrolysate as cosmetic agent according to claim 9, **characterised by** a reducing sugars content comprised between 25% and 45% by weight compared to the weight of dry matter of said hydrolysate.

11. Use of a locust bean gum hydrolysate as cosmetic agent according to any of claims 9 or 10, **characterised in that** the locust bean gum hydrolysate is an enzymatic hydrolysate.

12. Use of a locust bean gum hydrolysate as cosmetic agent according to claim 11, **characterised in that** it is an enzymatic hydrolysate obtained by an enzyme of mannase type.

13. Use of a locust bean gum hydrolysate as cosmetic agent according to any of claims 9 to 12, **characterised in that** the locust bean gum hydrolysate is a hydrolysate being in the form of a dry extract or a liquid extract.

14. Use of a locust bean gum hydrolysate as cosmetic agent according to any of claims 9 to 13 in a cosmetic composition, preferably a hair composition.

15. Use of a hair composition according to claim 8 to give a volume enhancing effect to hair.

16. Use of a hair composition according to claim 8 to coat hair while protecting it, advantageously for application on thin hair and/or hair without volume.

## Patentansprüche

1. Kosmetische Zusammensetzung, welche als Wirkstoff ein Hydrolysat von Johannisbrotgummi umfasst, welches durch einen Gehalt an reduzierenden Zuckern zwischen 10 Gew.-% und 60 Gew.-% eingeschlossen bezogen auf das Gewicht der Trockensubstanz des Hydrolysats gekennzeichnet ist.

2. Kosmetische Zusammensetzung nach Anspruch 1, **dadurch gekennzeichnet, dass** das Hydrolysat von Johannisbrotgummi einen Gehalt an reduzierenden Zuckern zwischen 25 Gew.-% und 45 Gew.-% bezogen auf das Gewicht der Trockensubstanz des Hydrolysats umfasst.

3. Kosmetische Zusammensetzung nach einem der Ansprüche 1 oder 2, **dadurch gekennzeichnet, dass** das Hydrolysat von Johannisbrotgummi ein enzymatisches Hydrolysat ist.

4. Kosmetische Zusammensetzung nach Anspruch 3, **dadurch gekennzeichnet, dass** es sich um ein enzymatisches Hydrolysat, das durch ein Enzym vom Typ Mannase erhalten wird, handelt.

5. Kosmetische Zusammensetzung nach einem der vorangegangenen Ansprüche, **dadurch gekennzeichnet, dass** das Hydrolysat von Johannisbrotgummi ein Hydrolysat ist, welches in Form eines Trockenauszugs oder eines flüssigen Extrakts vorliegt.

6. Kosmetische Zusammensetzung nach einem der vorangegangenen Ansprüche, welche eine Menge an Hydrolysat von Johannisbrotgummi zwischen 0,1 g und 2,0 g eingeschlossen pro 100 g der Zusammensetzung umfasst.

7. Kosmetische Zusammensetzung nach einem der vorangegangenen Ansprüche, welche in Form eines Shampoos, eines Gels, einer Lotion, eines Schaums, eines Sprays, einer Dispersion, eines Serums, einer Maske, einer Körpermilch, eines verstärkenden Balsams oder einer Creme vorliegt.

8. Für die Haare bestimmte Zusammensetzung nach einem der vorangegangenen Ansprüche.

9. Verwendung eines Hydrolysats von Johannisbrotgummi, welches durch einen Gehalt an reduzierenden Zuckern zwischen 10 Gew.-% und 60 Gew.-% eingeschlossen bezogen auf das Gewicht der Trockensubstanz des Hydrolysats gekennzeichnet ist, als kosmetisches Mittel.

10. Verwendung eines Hydrolysats von Johannisbrotgummi als kosmetisches Mittel nach Anspruch 9, welches durch einen Gehalt an reduzierenden Zuckern zwischen 25 Gew.-% und 45 Gew.-% eingeschlossen bezogen auf das Gewicht der Trockensubstanz des Hydrolysats gekennzeichnet ist.

11. Verwendung eines Hydrolysats von Johannisbrotgummi als kosmetisches Mittel nach einem der Ansprüche 9 oder 10, **dadurch gekennzeichnet, dass** das Hydrolysat von Johannisbrotgummi ein enzymatisches Hydrolysat ist.

12. Verwendung eines Hydrolysats von Johannisbrotgummi als kosmetisches Mittel nach Anspruch 11, **dadurch gekennzeichnet, dass** es sich um ein enzymatisches Hydrolysat, das durch ein Enzym vom Typ Mannase erhalten wird, handelt.

13. Verwendung eines Hydrolysats von Johannisbrotgummi als kosmetisches Mittel nach einem der Ansprüche 9 bis 12, **dadurch gekennzeichnet, dass** das Hydrolysat von Johannisbrotgummi ein Hydrolysat ist, welches in Form eines Trockenauszugs oder eines flüssigen Extrakts vorliegt.

14. Verwendung eines Hydrolysats von Johannisbrotgummi als kosmetisches Mittel nach einem der Ansprüche 9 bis 13 in einer kosmetischen, vorzugsweise für die Haare bestimmten Zusammensetzung.

15. Verwendung einer für die Haare bestimmten Zusammensetzung nach Anspruch 8, um den Haaren einen das Volumen erhöhenden Effekt zu verleihen.

16. Verwendung einer für die Haare bestimmten Zusammensetzung nach Anspruch 8, um die Haare zu umhüllen, indem sie geschützt werden, in vorteilhafter Weise für ein Auftragen auf feine und/oder kein Volumen aufweisende Haare.
